# FASCICULE DE BREVET EUROPEEN

(11) **EP 1 599 238 B1**
(45) Date de publication et mention de la délivrance du brevet: **14.06.2006**
(21) Numéro de dépôt: 04703194.3
(22) Date de dépôt: 19.01.2004
(51) Int. Cl.: A61L 27/34, A61L 27/54, A61F 2/08

(54) **LIGAMENT PROTHETIQUE BIOMIMETIQUE ET PROCEDE D'OBTENTION**
BIOMIMETISCHER BANDERSATZ UND HERSTELLUNGSVERFAHREN DAFÜR
BIOMIMETIC PROSTHETIC LIGAMENT AND PRODUCTION METHOD THEREOF

(30) Priorité: 17.01.2003 FR 0300495
(43) Date de publication de la demande: 30.11.2005
(73) Titulaire: L.A.R.S. - laboratoire d'application et de Recherche Scientifique, 21560 Arc-sur-Tille (FR)
(72) Inventeur: BRULEZ, Bernard, F-52400 Bourbonne-les-Bains (FR); LABOUREAU, Jacques-Philippe, F-06530 le Tignet (FR); MIGONNEY, Véronique, F-95600 Eaubonne (FR); CIOBANU, Mihaela, RO-6600 Iasi (RO); PAVON-DJAVID, Graciela, F-92800 Puteaux (FR); SIOVE, Alain, F-95230 Soisy sous Montmorency (FR)
(74) Mandataire: Honoré, Anne-Claire
(86) Numéro de dépôt international: PCT/FR2004/000103
(87) Numéro de publication internationale: WO 2004/067051

(56) Documents cités:
- EP-A- 0 727 233
- EP-A- 0 891 998
- US-A- 4 743 258
- US-A- 6 143 354
- US-B1- 6 200 626
- DEANTONIO P ET AL: "Free radical graft polymerization onto polymer substrates. 1. Method and quantification of active perioxide sites on polyethylene" POLYM PREPR DIV POLYM CHEM AM CHEM SOC; POLYMER PREPRINTS, DIVISION OF POLYMER CHEMISTRY, AMERICAN CHEMICAL SOCIETY AUG 1990 PUBL BY ACS, WASHINGTON, DC, USA, vol. 31, no. 2, août 1990 (1990-08), pages 448-449, XP009033307

## Description

La présente invention concerne un ligament prothétique biomimétique ainsi que son procédé d'obtention.

Les ligaments artificiels pour le remplacement de ligaments articulaires biologiques, et notamment ceux du genou, sont dans certains cas spécifiques préférés aux techniques de suture directe ou de reconstitution par greffe autogène. En effet, la qualité de ces ligaments tant sur le plan mécanique que celui de la biocompatibilité s'est considérablement améliorée ces vingt dernières années.

Ainsi par exemple, on connaît par le brevet français FR-96.14263, un ligament prothétique en polyéthylène téréphtalate (PET) dit à « fibres orientées ». Ce ligament est constitué d'une laize roulée ou pliée sur elle-même, laquelle laize comporte deux parties extrêmes intra-osseuses et une partie intermédiaire intra-articulaire constituée par un écheveau de fils de trame actifs longitudinaux, adjacents et non liés entre eux transversalement. Au montage du ligament, on donne une torsion longitudinale à chaque fil actif, aboutissant à un ligament dextrogyre ou lévogyre reproduisant le vrillage naturel des ligaments en flexion. Ce type de ligament en PET, matériau dont la biocompatibilité est bien connue, présente une résistance extrêmement élevée aux efforts de traction, flexion et torsion, laissant présumer une espérance de vie supérieure à celle des ligaments connus.

Toutefois, malgré ces énormes progrès sur le plan biomécanique, on constate encore chez les patients des ruptures de ces ligaments artificiels. Des études menées sur les causes de ces échecs ont mis en évidence une mauvaise repousse des fibroblastes après implantation. En effet, la distribution de ces cellules est inhomogène à la surface du ligament, les fibroblastes ne se développant pas dans les zones prothétiques intra-osseuses et se groupant en amas sur les fibres dans la zone intra-articulaire. En outre, ces fibroblastes présentent une morphologie arrondie anormale, avec peu de points d'ancrage à la surface du ligament. La sécrétion de collagène par ces cellules est de fait désordonnée et entraîne une individualisation des fibres de la zone intra-articulaire avec accumulation de tissus fibreux entre l'enveloppe et la partie centrale des prothèses, contribuant ainsi à leur défaillance.

Face à ce problème d'assurer une bonne « réhabitation » fibroblastique des prothèses, le brevet FR 2.651.994 propose d'imprégner le ligament prothétique de collagène, les fibres de collagène étant orientée selon l'axe longitudinal du ligament en vue d'obtenir une bonne direction de sa « réhabitation » par un tissu conjonctif vivant. Outre une efficacité limitée et peu satisfaisante, cette solution a pour inconvénient majeur de dépendre de la qualité difficilement reproductible du collagène utilisé.

La présente invention vise donc à remédier à ces problèmes en proposant un procédé de fonctionnalisation biomimétique de ligaments, notamment, procédé leur conférant notamment la capacité de mimer les matériaux vivants afin d'améliorer leur intégration biologique.

A cet égard et conformément à l'invention, il est proposé un procédé de fonctionnalisation biomimétique de prothèses en polyester, et notamment en polyéthylène téréphtalate (PET) remarquable en ce qu'il comporte une première étape de préparation de la surface en polyester en milieu solvant et une seconde étape de greffage de polymères ou copolymères biologiquement actifs à la surface en polyester desdites prothèses, laquelle étape de greffage consiste à réaliser une peroxydation de la surface par ozonation suivie d'une étape de polymérisation radicalaire d'une solution d'au moins un monomère. On notera que par prothèse en polyester, on entend toute prothèse constituée de fibres de PET ou de dérivé de polyester donnant des performances convenables pour son utilisation dans des ligaments ainsi que toute prothèse présentant à sa surface ou celle de ses fibres une couche de polyester et notamment de PET.

On comprend bien qu'en créant à la surface d'un ligament fabriqué selon le brevet FR-96.14263 susmentionné un « tapis » de polymères biologiquement actif , on modifie ainsi ses propriétés d'adhérence et de prolifération cellulaire, les rendant favorables à une réhabitation fibroblastique normale et homogène, comme le montrent les résultats de culture cellulaire obtenus.

On peut améliorer ces propriétés en procédant, à l'étape de préparation de la surface en polyester en milieu solvant seulement, ou en milieu solvant puis en milieu aqueux, qui précède l'étape de greffage.

Ces deux variantes de cette étape de préparation contribuent considérablement à améliorer l'efficacité de l'étape de greffage, en augmentant la qualité de la surface greffée ainsi que la quantité de (co)polymères greffés. Elles jouent également un rôle non négligeable dans le caractère biocompatible du ligament ainsi traité.

D'autres avantages et caractéristiques ressortiront mieux de la description qui va suivre, de la variante d'exécution complète, donnée à titre d'exemple non limitatif, du procédé de fonctionnalisation selon l'invention.

Le procédé selon l'invention est ici appliqué à des prothèses ligamentaires déjà formées ou encore aux trames de tissu en polyester entrant dans la fabrication desdites prothèses : le procédé se décompose selon les étapes suivantes :

### Etape 1 : Préparation de la surface en polyester

### 1A) En milieu solvant :

Cette étape dite de désensimage est nécessaire afin d'éliminer les graisses et impuretés incorporées lors de la fabrication de la trame en polyester servant de structure au ligament. Elle permet ainsi d'éviter des réactions pathologiques de type synovite aiguë lors de l'implantation chez le patient. En outre, cette étape permet d'assurer une croissance des fibroblastes sur une surface en polyester ainsi nettoyée, croissance non observée sur des surfaces non nettoyées.

On distingue deux variantes de cette préparation en milieu solvant, selon la nature du solvant choisi.

### Variante 1: Désensimage sans gonflement de la surface :

On applique un minimum de 12 cycles d'extraction dans un appareil de SOHXLET et un contrôle des résidus de corps gras après le 12ème cycle de nettoyage à l'hexane. On fait suivre ces cycles de nettoyage à l'hexane par des cycles de lavage à l'éther éthylique (RPE), trois lavages minimum avec contrôle des résidus après le troisième lavage.

### Variante 2 : Désensimage avec un solvant apte à gonfler la surface en polyester :

L'utilisation d'un solvant apte à gonfler la surface du polyester offre l'avantage d'améliorer le greffage en augmentant le nombre de peroxydes sur la surface traitée lors de l'étape d'ozonation.

Un tel solvant a pour caractéristique d'avoir une constante diélectrique supérieure à 30. De préférence, ce solvant est de type éther cyclique ou aliphatique. En outre, on le choisira dans le groupe de solvant suivant : tétrahydrofuranne (THF), diméthylsulfoxyde (DMSO), N,N-diméthylformamide (DMF), N,N-diméthylacétamide (DMA), N-méthylpyrrolidone (NMP). Ces solvants ont l'avantage de présenter une toxicité faible ou nulle et permettent ainsi une utilisation facile en milieu industriel.

Le traitement se fait par immersion des pièces en polyester dans le solvant pendant une durée de l'ordre de 5 minutes à une heure environ, de préférence en 10 à 25 minutes. Ainsi, à titre d'exemple, on choisira une durée de 15 minutes à température ambiante pour une immersion dans du tétrahydrofuranne (THF).

### 1B) En milieu aqueux :

Le but de cette étape optionnelle de préparation de la surface en milieu aqueux est d'éliminer les résidus de fabrication du polyester présent à sa surface, tels que des acides organiques de faible poids moléculaire comme par exemple, l'acide téréphtalique pour le PET. On obtient ainsi une surface parfaitement apprêtée avant ozonation.

Le traitement consiste à laver les pièces en polyester dans une solution de carbonate de sodium (Na₂CO₃) à 5% en poids dans l'eau distillée. Ce lavage est effectué à chaud, c'est-à-dire à plus de 60°C et moins de 120°C et de préférence à légère ébullition, soit 100°C ± 5°C, pendant une dizaine de minutes. Bien entendu, tout autre carbonate alcalin ou alcalino-terreux tel que K₂CO₃ ou CaCO₃ peut être utilisé. Le lavage est suivi de rinçages successifs à l'eau distillée jusqu'à ce que le pH de l'eau de rinçage soit revenu à 7.

### 1C) Nettoyage :

Quelles que soient les étapes précédemment effectuées (variante 1 ou 2 de l'étape 1A, puis l'étape 1B, ou seulement l'étape 1A), le produit en polyester est ensuite nettoyé, par exemple par un rinçage à l'éthanol absolu ou au tétrahydrofuranne (THF) suivi d'un séchage en étuve d'une durée de 30 minutes, par exemple.

### Etape 2 : Greffage de polymères ou de copolymères biologiquement actifs sur la surface en polyester :

### 2A) Choix et préparation des monomères :

Les monomères utilisés selon l'invention sont des monomères susceptibles de polymérisation et de copolymérisation radicalaire donnant naissance à des polymères bio-compatibles stimulant la prolifération et la différenciation cellulaire et plus particulièrement celle des fibroblastes. De tels monomères contenant des groupes carboxylate, phosphate, sulfonate et sulfate sont, par exemple, décrits dans le brevet US 6.365.692 et peuvent être utilisés selon l'invention seuls ou en mélange. On préférera utiliser de l'acide méthacrylique et du styrène sulfonate, ainsi que leurs mélanges. Avant de les utiliser pour la polymérisation, ces monomères seront purifiés au préalable. Ainsi par exemple, pour du styrène sulfonate de sodium, on le purifie par recristallisation dans un mélange d'eau bidistillée/alcool (10/90,v:v), puis on le dissout à 70°C dans cette solution. On le filtre ensuite sous vide avec un disque en verre fritté d'indice de porosité 3 et on le conserve à 4°C. Les cristaux de sulfonate de sodium formés sont récupérés par filtration et le solide obtenu est séché sous vide à 50°C jusqu'à l'obtention d'un poids constant.

### 2B) Ozonation :

Les prothèses ou les trames en polyester constitutives de ces prothèses préalablement traitées selon l'étape 1 sont introduites dans un dispositif d'ozonation tel que classiquement utilisé.

Par exemple, on pourra utiliser un réacteur tubulaire de 500 cm3 contenant 100 cm3 d'eau bidistillée, lequel réacteur est muni d'un tube plongeur d'alimentation en ozone. On pourra, par exemple, utiliser un flux gazeux équivalent en ozone à 50 g/m3 d'oxygène. Pour une telle quantité d'ozone, la durée optimale d'ozonation du PET est de 20 à 90 minutes. Les mesures du taux de peroxyde montre que le taux optimal est obtenu entre 30 et 60 minutes d'ozonation, toujours pour ce même flux d'ozone. On notera également qu'une durée d'ozonation supérieure à 90 minutes dégrade fortement la surface en PET.

On relèvera par ailleurs l'apport de la variante 2 de l'étape 1A. En effet, l'utilisation d'un solvant apte à gonfler la surface augmente le taux de peroxyde d'un facteur 5, par rapport à l'utilisation d'un solvant sans gonflement. Une fois l'ozonation terminée, les prothèses ou les trames en polyester introduites dans le dispositif d'ozonation sont rincées et nettoyées, par exemple selon le protocole suivant : rincer trois fois à l'eau bidistillée, puis trois fois à l'alcool absolu, puis trois fois au tétrahydrofuranne (THF). Ensuite, sécher à l'étuve à vide pendant 30 minutes à 25°C.

### 2C) Polymérisation :

Le ou les monomères choisis et préparés selon l'étape 2A sont mis en solution dans de l'eau, de préférence bidistillée. On peut choisir toute concentration compatible avec la mise en oeuvre de la réaction de polymérisation radicalaire avec un minimum de 5% en poids. On choisira avantageusement des concentrations proches de la solubilité limite du ou des monomère (s) dans la solution, avec un milieu visqueux favorisant ainsi des réactions de propagation polymérique radicalaire par rapport à des réactions de terminaison : on obtient ainsi des chaînes polymériques greffées de plus grande taille. Cela revient à choisir une concentration pondérale k = s - ∈, ou s est la solubilité limite et ∈ vaut 1 à 7% en poids. Ainsi par exemple, dans le cas du polystyrène sulfonate dont la limite de solubilité est de 20% en poids, on choisira une concentration de 15%.

La durée de l'étape de polymérisation dépend de la nature du monomère. Elle est estimée au temps nécessaire à la gélification du milieu à la température de réaction. Ainsi, par exemple, on retiendra pour le polystyrène sulfonate qu'à 70°C, la polymérisation durera 1 heure et qu'à 65°C, elle durera 1 heure 30.

La réaction de polymérisation est conduite dans une enceinte hermétiquement fermée et débarrassée de tout oxygène, par exemple, en réalisant un bullage à l'argon. On introduit dans cette enceinte la solution de monomères ou de comonomères que l'on veut faire réagir et les prothèses ou bandes de tissus en polyester préalablement ozonées. Le récipient hermétiquement fermé est chauffé au bain-marie à la température et à la durée déterminées comme exposé précédemment.

En fin de réaction, les éléments en polyester qui ont été greffés sont extraits du réacteur. On peut ensuite laver ces matériaux greffés afin d'éliminer notamment des résidus de monomère(s) qui n'ont pas réagit. Par exemple, on peut laver la surface fonctionnalisée plusieurs fois avec un solvant adéquat du ou des monomère (s), de l'eau bidistillée par exemple, et on peut optionnellement finir le lavage avec tout solvant adéquat, de l'éthanol absolu par exemple, afin d'éliminer d'éventuelle traces de polymères non greffés.

### Etape 3 : Imprégnation avec des agents biochimiques :

Cette étape est optionnelle. Elle vise à renforcer la capacité d'intégration biologique du ligament auquel on a précédemment greffé des polymères biomimétiques tel qu'exposé aux étapes 1 et 2. Ainsi, l'imprégnation de la prothèse par un ou plusieurs agents biochimiques vise à augmenter ses propriétés d'adhérence et de prolifération cellulaire. Ces agents biochimiques favorisant la colonisation par des fibroblastes sont une protéine de la famille des fibronectines et/ou du collagène de type I et/ou III. On utilisera avantageusement un mélange des précédentes protéines, c'est-à-dire un mélange de fibronectines et collagène de type I et/ou III : on observe un effet synergique sur l'adhérence des fibroblastes. L'imprégnation de la prothèse par ces agents pourra, par exemple, être effectuée par trempage dans un bain contenant du collagène.

Il va de soi que cette étape d'imprégnation ne suit pas forcément l'étape de greffage et qu'elle peut s'insérer et s'intercaler entre d'autres étapes de préparation du ligament en fonction de son stade de fabrication. En outre, on fera avantageusement suivre cette étape d'imprégnation par une étape de stérilisation du ligament.

Afin de mesurer l'efficacité du procédé qui vient d'être décrit, des essais comparatifs ont été menés sur des portions de tissus en fibres de PET tricotées, le polyester étant semi-cristalin avec un taux de cristallinité de 40% C ± 5% et ayant une température de fusion proche de 260°C ± 5°C. Ces tissus en fibre sont constitutifs de ligaments artificiels tels que fabriqués et commercialisés par la demanderesse. On a ainsi préparé des fibres de PET témoin non greffées et deux types de fibres de PET greffées par du poly(styrène sulfonate de sodium) et greffées également par un mélange équipondéral d'acide méthacrylique (AM) et de styrène sulfonate de sodium (SSNa). On trouvera au tableau 1 les différentes conditions de préparation.

**Tableau 1 : Préparation des échantillons**

| | **Préparation de la surface** | **Ozonation** | **Greffage** |
|---|---|---|---|
| **Fibre PET non greffées** | Hexane et éther éthylique ;Na₂CO₃ à 5 % | - | - |
| **Fibres PET greffées (1)** | THF: 15 min; Na₂CO₃: **5%** | 60 min à 50g O₃/m³ 0₂ 50g O₃/m³ 0₂ | Solution de SSNa 15% en poids ; 1h30 à en poids ; 1h30 à 65°C |
| **Fibres PET greffées (2)** | THF : 15 min ; Na₂CO₃: **5%** | 60 min à 50g 0₃/m³ 0₂ | Solution à 15%SSNa et 15% AM (en poids) ; 1h30 à 65°C |

On a ensuite procédé à différents tests d'évaluation biologique et notamment de cultures de fibroblastes et d'adhérence de fibroblastes sur ces différentes surfaces et les résultats obtenus figurent dans le tableau ci-après :

**Tableau 2 :Evaluation biologique**

| **Paramètres** | **PET non greffé** | **PET greffé (1) et (2)** |
|---|---|---|
| **Nombre de cellules à confluence (culture dans des plaques de 24 puits)** | 1.10⁵ cellules/puit | 1.10⁵ cellules/puit |
| **Développement des fibroblastes** | Répartition en amas inhomogène le long des fibres | Répartition homogène sur toute la surface du tissu |
| **Morphologie** | Forme arrondie | Forme allongée et étalée |
| **Force d'adhérence (unité relative)** | 1 | 5-8 |
| **Adsorption de collagène de type I** (proportions relatives en ng/cm²) | 100 | 143 |

On observe donc que le greffage de polymères biomimétiques, et en particulier de styrène sulfonate à la surface de ligaments en polyester permet un développement normal et homogène des fibroblastes sur cette surface.

On relèvera en outre que la surface traitée présente une affinité plus élevée vis-à-vis des protéines ce qui permet d'avoir une meilleure adsorption de collagène et d'autres facteurs biochimiques favorisant la colonisation par des fibroblastes. On comprend donc bien que des ligaments, dont la surface aura été traitée selon les étapes 1 et 2 du procédé, et auxquels on fait ensuite subir l'étape 3 d'imprégnation par de tels facteurs biochimiques, verront encore leurs capacités à faire adhérer les fibroblastes et à orienter correctement leur développement, nettement améliorées.

Enfin, il va de soi que le procédé de fonctionnalisation biomimétique selon l'invention peut être appliqué à tout type de prothèse en polyester pour lesquelles on veut améliorer l'intégration biologique et que les exemples que l'on vient de donner ne sont que des illustrations particulières en aucun cas limitatives des domaines d'application de l'invention.

## Revendications

1. Procédé de traitement de prothèses artificielles en polyester, et notamment en polyéthylène téréphtalate, afin de leur conférer la capacité de mimer les matériaux vivants et spécialement de faciliter l'ancrage et le développement des fibrolastes après l'implantation desdites prothèses, dit procédé de fonctionnalisation biomimétique **caractérisé en ce qu'**il comporte une première étape de préparation de la surface en polyester en milieu solvant suivie d'une étape de greffage de polymères ou copolymères biologiquement actifs à la surface en polyester desdites prothèses, laquelle étape de greffage consiste à réaliser une peroxydation de la surface par ozonation suivie d'une polymérisation radicalaire d'une solution d' au moins un monomère.

2. Procédé selon la revendication 1 **caractérisé en ce que** le polyéthylène téréphtalate est semi-cristallin.

3. Procédé selon l'une quelconque des revendications 1 ou 2 **caractérisé en ce que** la durée d'ozonation pour une teneur d'ozone de l'ordre de 50g/cm3 est comprise entre 20 et 90 min.

4. Procédé selon l'une quelconque des revendications 1 à 3 **caractérisé en ce que** le monomère est du styrène sulfonate de sodium

5. Procédé selon l'une quelconque des revendications 1 à 4 **caractérisé en ce que** la solution de monomères a une concentration en monomère s) comprise entre 5% et k%, où k est une concentration proche de la solubilité limite du ou des monomère(s)dans la solution.

6. Procédé selon l'une quelconque des revendications précédentes **caractérisé en ce que** la première étape de préparation de la surface en polyester en milieu solvant est suivie par une étape supplémentaire de préparation en milieu aqueux avant de procéder au greffage.

7. Procédé selon l'une quelconque des revendications précédentes **caractérisé en ce que** le milieu solvant est constitué d'hexane et d'éther éthylique.

8. Procédé selon l'une quelconque des revendications 1 à 6 **caractérisé en ce que** le milieu solvant est constitué par au moins un solvant apte à modifier la surface par gonflement.

9. Procédé selon la revendication 8 **caractérisé en ce que** le solvant apte à modifier la surface par gonflement est du type éther cyclique ou aliphatique ayant une toxicité faible ou nulle.

10. Procédé selon l'une quelconque des revendications 8 ou 9 **caractérisé en ce que** le solvant apte à modifier la surface par gonflement a une constante diélectrique supérieure à 30.

11. Procédé selon l'une quelconque des revendications 8, 9 ou 10 **caractérisé en ce que** le solvant apte à modifier la surface par gonflement est choisi dans le groupe de solvants suivants : tétrahydrofuranne (THF), diméthylsulfoxyde (DMSO), N,N-diméthylformamide (DMF), N,N-diméthylacétamide (DMA), N-méthylpyrrolidone (NMP).

12. Procédé selon l'une quelconque des revendications 6 à 11 **caractérisé en ce que** l'étape de préparation de la surface en milieu aqueux consiste à traiter la surface en polyester à chaud avec une solution aqueuse de sels de carbonates alcalins ou alcalino-terreux, comme par exemple Na₂CO₃ ou CaCO₃, afin d'éliminer les résidus de fabrication du polyester présents à sa surface.

13. Procédé selon l'une quelconque des revendications précédentes **caractérisé en ce qu'**il comporte une étape supplémentaire d'imprégnation de la prothèse après l'étape de greffage par un ou plusieurs agents biochimiques favorisant la colonisation par des fibroblastes.

14. Procédé selon la revendication 13 **caractérisé en ce que** l'agent biochimique est une protéine de la famille des fibronectines et/ou du collagène de type I et/ou III.

15. Ligament artificiel en fibres tissées ou tricotées de PET **caractérisé en ce que** lesdites fibres ont été traitées selon le procédé conforme à l'une quelconque des revendications 1 à 14.

## Claims

1. A method for treating artificial prostheses in polyester, and notably in polyethylene terephtalate, so as to provide them with the capability of mimicking living materials and particularly to facilitate anchoring and development of fibroblasts after implantation of said prostheses, a so-called biomimetic functionalization method, **characterized in that** it includes a first step for preparing the polyester surface in a solvent medium by a step for grafting polymers or copolymers biologically active at the polyester surface of said protheses, which grafting step consists of achieving peroxidation of the surface by ozonation followed by radical polymerization of a solution of at least one monomer.

2. The method according to claim 1, **characterized in that** the polyethylene terephtalate is semi-crystalline.

3. The method according to any of claims 1 or 2, **characterized in that** the ozonation time for an ozone content of the order of 50 g/cm³ is between 20 and 90 min.

4. The method according to any of claims 1 to 3, **characterized in that** the monomer is sodium styrene sulfonate.

5. The method according to any of claims 1 to 4, **characterized in that** the solution of monomers has a concentration of monomer(s) between 5% and k%, where k is a concentration close to the limiting solubility of the monomer (s) in the solution.

6. The method according to any of the preceding claims, **characterized in that** the first step for preparing the polyester surface in a solvent medium is followed by an additional preparation step in an aqueous medium before proceeding with grafting.

7. The method according to any of the preceding claims, **characterized in that** the solvent medium consists of hexane and ethyl ether.

8. The method according to any of claims 1 to 6, **characterized in that** the solvent medium is formed with at least one solvent capable of altering the surface by swelling.

9. The method according to claim 8, **characterized in that** the solvent capable of altering the surface by swelling is of the aliphatic or cyclic ether type with low or zero toxicity.

10. The method according to any of claims 8 or 9, **characterized in that** the solvent capable of altering the surface by swelling has a dielectric constant larger than 30.

11. The method according to any of claims 8, 9 or 10, **characterized in that** the solvent capable of altering the surface, by swelling is selected from the group of following solvents: tetrahydrofurane (THF), dimethylsulfoxide (DMSO), N,N-dimethylformamide (DMF), N,N-dimethylacetamide (DMA), N-methylpyrrolidone (NMP).

12. The method according to any of claims 6 to 11, **characterized in that** the step for preparing the surface in an aqueous medium consists of hot-treating the polyester surface with an aqueous solution of alkali metal or alkaline earth carbonate salts, such as for example Na₂CO₃ or CaCO₃, in order to remove manufacturing residues from the polyester, present at its surface.

13. The method according to any of the preceding claims, **characterized in that** it includes an additional step for impregnating the prosthesis after the grafting step with one or more biochemical agents promoting colonization by fibroblasts.

14. The method according to claims 13, **characterized in that** the biochemical agent is a protein from the family of fibronectins and/or collagen of type I and/or III.

15. An artificial ligament in woven or knitted PET fibers, **characterized in that** said fibers were treated according to the method in accordance with any of claims 1 to 14.

## Patentansprüche

1. Verfahren zur Behandlung von künstlichen Prothesen aus Polyester, und vor allem aus Terephthalatpolyester, um ihnen die Fähigkeit zu verleihen, lebende Materialien nachzuahmen und speziell die Verankerung und die Entwicklung der Fibroblasten nach Implantation der besagten Prothesen zu erleichtern, wobei dieses biomimetische Funktionalisierungsverfahren **dadurch gekennzeichnet ist, dass** es einen ersten Schritt zur Vorbereitung der Polyesteroberfläche in einem Lösungsmedium umfasst, dem ein Schritt der Verpflanzung von biologisch aktiven Polymeren oder Copolymeren auf die Polyesteroberfläche der besagten Prothesen folgt, wobei dieser Verpflanzungsschritt darin besteht, eine Peroxidation der Oberfläche durch Ozonierung durchzuführen, gefolgt von einer Radikalpolymerisation einer Lösung mindestens eines Monomers.

2. Verfahren nach Anspruch 1, **dadurch gekennzeichnet, dass** das Terephthalatpolyester halbkristallin ist.

3. Verfahren nach einem der Ansprüche 1 oder 2, **dadurch gekennzeichnet, dass** die Dauer der Ozonierung für einen Ozongehalt in der Größenordnung von 50 g/cm³ 20 bis 90 Minuten beträgt.

4. Verfahren nach einem der Ansprüche 1 bis 3, **dadurch gekennzeichnet, dass** das Monomer Natriumstyrensulfonat ist.

5. Verfahren nach einem der Ansprüche 1 bis 4, **dadurch gekennzeichnet, dass** die Monomerlösung eine Monomerkonzentration von 5 % bis k % hat, wobei k eine Konzentration nahe der Grenzlöslichkeit des Monomers oder der Monomere in der Lösung ist.

6. Verfahren nach einem der vorhergehenden Ansprüche, **dadurch gekennzeichnet, dass** dem ersten Schritt der Vorbereitung der Polyesteroberfläche in einem Lösungsmedium ein zusätzlicher Schritt der Vorbereitung in wässrigem Medium vor der Durchführung der Verpflanzung folgt.

7. Verfahren nach einem der vorhergehenden Ansprüche, **gekennzeichnet**, **dass** das Lösungsmedium aus Hexan und Ethylether besteht.

8. Verfahren nach einem der Ansprüche 1 bis 6, **dadurch gekennzeichnet, dass** das Lösungsmedium aus mindestens einem Lösungsmittel besteht, das in der Lage ist, die Oberfläche durch Aufquellen zu verändern.

9. Verfahren nach Anspruch 8, **dadurch gekennzeichnet, dass** das Lösungsmittel, das die Oberfläche durch Aufquellen verandern kann, vom Typ eines zyklischen oder aliphatischen Ethers ist mit einer schwachen oder keiner Toxizität.

10. Verfahren nach einem der Ansprüche 8 oder 9, **dadurch gekennzeichnet, dass** das Lösungsmittel, das die Oberfläche durch Aufquellen verändern kann, eine dielektrische Konstante von über 30 hat.

11. Verfahren nach einem der Ansprüche 8, 9 oder 10, **dadurch gekennzeichnet, dass** das Lösungsmittel, das die Oberfläche durch Aufquellen verändern kann, aus der Gruppe der folgenden Lösungsmittel ausgewählt ist: Tetrahydrofuran (THF), Dimethylsulfoxid (DMSO), N,N-Dimethylformamid (DMF), N,N-Dimethylacetamid (DMA), N-Methylpyrrolidon (NMP).

12. Verfahren nach einem der Ansprüche 6 bis 11, **dadurch gekennzeichnet, dass** der Schritt der Vorbereitung der Oberfläche in wässrigem Medium darin besteht, die Polysteroberfläche warm mit einer wässrigen Lösung von Salzen alkalischer Karbonate oder von Alkalinerde-Karobonaten zu behandeln, wie zum Beispiel Na₂CO₃ oder CaCO₃, um die auf ihrer Oberfläche vorhandenen Reste der Polyesterherstellung zu entfernen.

13. Verfahren nach einem der vorhergehenden Ansprüche, **dadurch gekennzeichnet, dass** es einen zusätzlichen Schritt der Imprägnierung der Prothese nach dem Schritt der Verpflanzung durch ein oder mehrere biochemische/s Mittel umfasst, welche/s die Besiedlung durch Fibroblasten begünstig/en/t.

14. Verfahren nach Anspruch 13, **dadurch gekennzeichnet, dass** das biochemische Mittel ein Protein der Familie der Fibronectine und / oder von Kollagen des Typs I und / oder III ist.

15. Künstliches Band aus gewebten oder gewirkten PET-Fasern, **dadurch gekennzeichnet, dass** die besagten Fasern gemäß dem Verfahren nach einem der Ansprüche 1 bis 14 behandelt worden sind.
